## Europäisches Patentamt
## European Patent Office
## Office européen des brevets

(19)

(11) Veröffentlichungsnummer: **0 008 752**
**B1**

(12) **EUROPÄISCHE PATENTSCHRIFT**

(45) Veröffentlichungstag der Patentschrift:
12.08.81

(51) Int. Cl.³: **C 07 D 307/79**

(21) Anmeldenummer. **79103127.1**

(22) Anmeldetag: **24.08.79**

(54) Verfahren zur Herstellung von 2,3-Dihydro-2-äthylbenzofuran-5-ylessigsäure.

(30) Priorität: **05.09.78 IT 352078**

(43) Veröffentlichungstag der Anmeldung:
**19.03.80 Patentblatt 80/6**

(45) Bekanntmachung des Hinweises auf die Patenterteilung:
**12.08.81 Patentblatt 81/32**

(84) Benannte Vertragsstaaten:
**AT CH DE NL SE**

(56) Entgegenhaltungen:
**GB-A-1 483 061**
**JOURNAL OF THE CHEMICAL SOCIETY,**
**1952,**
**London, GB**
**N. M. CULLINANE et al.: »Titanium tetrachloride as a catalyst in the Friedel-crafts reaction, Part I Acylation«, Seiten 376—380**

(73) Patentinhaber: **ALFA FARMACEUTICI S.p.A., Via Ragazzi del '99 no 5, I-40133 Bologna (IT)**

(72) Erfinder: **Tamagnone, Gianfranco, Dr., Via Ronzani no 55, I-40033 Casalecchio di Reno (Bologna) (IT)**

(74) Vertreter: **Beszédes, Stephan G., Dr., Am Heideweg 2 Postfach 1168, D-8060 Dachau (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**0 008 752**

Verfahren zur Herstellung von 2,3-Dihydro-2-äthylbenzofuran-5-ylessigsäure

Die Erfindung betrifft ein neues Verfahren zur Herstellung von 2,3-Dihydro-2-äthylbenzofuran-5-ylessigsäure.

In der britischen Patentschrift 1 483 061 sind die pharmakologischen Eigenschaften und Verfahren zur Herstellung einer Klasse von Verbindungen, welche als nicht-steroide entzündungshemmende Mittel von beträchtlichem Interesse sind, beschrieben. Unter den beschriebenen Verbindungen ist auch die 2,3-Dihydro-2-äthylbenzofuran-5-ylessigsäure. Eines der zur Herstellung dieser Verbindung angegebenen Verfahren umfaßt die Acylierung des 2,3-Dihydro-2-äthylbenzofuranes mit Chlorglyoxylsäureäthylester (mit anderer Bezeichnung Oxalylmonochloridmonoäthylester) in Gegenwart von wasserfreiem Aluminiumchlorid (Aluminium trichlorid) in Nitrobenzol, anschließende Hydrolyse des erhaltenen Esters mit Natriumhydroxyd zur entsprechenden Ketosäure und schließlich Reduktion der Ketogruppe mit Hydrazinhydrat in Äthylenglykol.

Es wurde jedoch festgestellt, daß dieses Verfahren erhebliche Nachteile, welche besonders bei der technischen bzw. industriellen Herstellung des gewünschten Produktes zutage treten, hat. Eines derselben besteht in der Tatsache, daß bei Verwendung des Aluminiumchlorides als Friedel-Crafts-Katalysator bei der Acylierungsreaktion des 2,3-Dihydro-2-äthylbenzofuranes und der Hydrolyse des als Zwischenprodukt erhaltenen Aluminiumkomplexes es notwendig ist, die Temperatur innerhalb sehr enger Grenzen, im allgemeinen auf −5 bis 0°C, zu halten. Über 0°C erfolgt in beträchtlichem Maße die Bildung von unerwünschten Nebenprodukten, welche außer der Herbeiführung von Schwierigkeiten bei der Reinigung des Produktes zur Verminderung der Ausbeute an ihm beitragen. Es wurde nämlich festgestellt, daß unter solchen Bedingungen sich schwer entfernbare Chormanderivate bilden können (Boll. Chim. Farm. 116 [1977], Seite 81). Sowohl die Acylierungsreaktion als auch die anschließende Hydrolyse des Aluminiumkomplexes sind stark exotherme Reaktionen und daher ist es sehr schwierig, die Temperatur innerhalb der oben angegebenen Grenzen zu halten. Die Verwendung des Aluminiumchlorides bringt die Verwendung von Nitrobenzol als Reaktionsmedium mit sich: dieses Lösungsmittel muß dann mittels Wasserdampfdestillation entfernt werden. Auch diese Trennung, welche bei hoher Temperatur und während langer Zeiten durchgeführt wird, trägt zur Verminderung der Ausbeuten bei, indem sie unerwünschte Zersetzungsreaktionen hervorruft. Überdies ist das im genannten bekannten Verfahren verwendete Nitrobenzol ziemlich toxisch.

Ferner ist aus Journal of the Chemical Society 1952, Seiten 376 bis 380 die Acylierung von Benzol, Toluol und Anisol mit den Carbonsäurechloriden Acetylchlorid, n-Propionylchlorid und n-Butyrylchlorid sowie Benzoylchlorid in Gegenwart von Titantetrachlorid als Friedel-Crafts-Katalysator ohne Lösungsmittel, wobei die zu acylierenden Verbindungen Benzol, Toluol bzw. Anisol im Überschuß verwendet werden, bekannt. Auf Seite 376, Zeile 6 von unten bis 5 von unten dieser Druckschrift ist darauf hingewiesen, daß Titantetrachlorid als Friedel-Crafts-Katalysator weniger wirksam als Aluminiumchlorid ist. Ferner ist auf Seite 377, drittletzte bis vorletzte Zeile der genannten Druckschrift bemerkt, daß die Carbonsäureanhydride reinere Produkte bei weniger Teerbildung ergeben als die Carbonsäurechloride. Durch dieses Verfahren werden aber nur verhältnismäßig geringe Ausbeuten an Acylierungsprodukten erhalten, indem die höchste Ausbeute bei Verwendung eines aliphatischen Carbonsäurechlorides (im Falle der Acylierung von Anisol mit n-Butyrylchlorid zu p-Methoxy-n-butyrophenon) nur 44,5%, bezogen auf die der Acylierung unterworfene Ausgangssubstanz (Anisol), war, wobei im Falle der Acylierung von Benzol mit Acetylchlorid die noch viel geringere Ausbeute von nur 3,8% am Acetylierungsprodukt Acetophenon, bezogen auf das eingesetzte Benzol, erzielt werden konnte, wie es sich aus den in der Zusammenstellung auf Seite 378 der genannten Druckschrift angegebenen Ausbeutewerten, die alle auf das Acylierungsmittel (also nicht auf die der Acylierung unterworfene Ausgangssubstanz) bezogen sind (vergleiche Seite 379, Zeile 23 von unten bis 21 von unten der genannten Druckschrift), durch Umrechnung auf die jeweilige der Acylierung unterworfene Ausgangssubstanz ergibt.

Der Erfindung liegt die Aufgabe zugrunde, unter Behebung der Nachteile des Standes der Technik ein besseres und einfacheres Verfahren zur Herstellung von 2,3-Dihydro-2-äthylbenzofuran-5-ylessigsäure, durch welches höhere Ausbeuten an dieser erzielt werden können, bei welchem in leicht entfernbaren und weniger toxischen Lösungsmitteln gearbeitet werden kann und Friedel-Crafts-Katalysatoren, die im Reaktionsmedium gut löslich sind und daher schon in verhältnismäßig geringen Mengen die Durchführung der Umsetzung in Lösung in homogener Phase und somit eine gute Verteilung und Ableitung der Reaktionswärme ermöglichen, verwendet werden können und mit welchem innerhalb eines ziemlich weiten Temperaturbereiches ohne die Bildung von unerwünschten Nebenprodukten gearbeitet werden kann, zu schaffen.

Gegenstand der Erfindung ist ein Verfahren zur Herstellung von 2,3-Dihydro-2-äthylbenzofuran-5-ylessigsäure durch Umsetzen von im wesentlichen äquimolaren Mengen von Chlorglyoxylsäureäthylester und 2,3-Dihydro-2-äthylbenzofuran in Gegenwart eines Friedel-Crafts-Katalysators in einem Lösungsmittel bei niederer Temperatur sowie im weiteren unter Verwendung einer Umsetzung mit Hydrazinhydrat als Reaktionsteilnehmer, welches dadurch gekennzeichnet ist, daß für die Umsetzung des Chlorglyoxylsäureäthylesters mit dem 2,3-Dihydro-2-äthylbenzofuran als Friedel-Crafts-Katalysator Titantetrachlorid und/oder Zinntetrachlorid und als Lösungsmittel 1 oder mehr niedere aliphatische Chlorkohlenwasserstoffe eingesetzt werden und als Temperatur −30 bis +50°C angewandt wird sowie der aus der Reaktionsmischung isolierte erhaltene 2,3-Dihydro-2-äthylbenzofuran-5-ylglyoxylsäureäthylester unmittelbar mit 80 bis 100%igem Hydrazinhydrat in Gegenwart von 1 oder mehr Alkoholen mit 1 bis 6 Kohlenstoffatomen und/oder Glykolen mit 2 bis 4 Kohlenstoffatomen als Lösungsmittel(n) zum 2,3-Dihydro-2-äthylbenzofuran-5-ylglyoxylsäurehydrazidhydrazon umgesetzt wird und das letztere unmittelbar in Gegenwart einer Lösung von 1 oder mehr starken anorganischen Basen auf eine Temperatur von 100 bis 200° C erhitzt wird.

Die erfindungsgemäße Festlegung von »im wesentlichen« äquimolaren Mengen des Chlorglyoxylsäureäthylesters und 2,3-Dihydro-2-äthylbenzofuranes umfaßt auch von den äquimolaren Mengen etwas abweichende Mengen. So ist es vorteilhaft, für die Umsetzung des Chlorglyoxylsäureäthylesters mit dem 2,3-Dihydro-2-äthylbenzofuran einen leichten Überschuß des Chlorglyoxylsäureäthylesters von 1,05 bis 1,3 Mol je Mol 2,3-Dihydro-2-äthylbenzofuran zu verwenden. Ein Beispiel hierfür ist die Verwendung von 1,2 Mol Chlorglyoxylsäureäthylester je Mol 2,3-Dihydro-2-äthylbenzofuran.

Bevorzugt wird als Katalysator Titantetrachlorid verwendet.

Zweckmäßig wird für die Umsetzung des Chlorglyoxylsäureäthylesters mit dem 2,3-Dihydro-2-äthylbenzofuran der Friedel-Crafts-Katalysator in einer Menge von 1 bis 1,2 Mol je Mol 2,3-Dihydro-2-äthylbenzofuran verwendet.

Vorteilhaft wird für die Umsetzung des Chlorglyoxylsäureäthylesters mit dem 2,3-Dihydro-2-äthylbenzofuran als Lösungsmittel Methylenchlorid, Chloroform, Tetrachlorkohlenstoff, Dichloräthan oder Tetrachloräthan oder eine Mischung derselben verwendet.

Niedrigere Temperaturen als die erfindungsgemäß für die Umsetzung des Chlorglyoxylsäureäthylesters mit dem 2,3-Dihydro-2-äthylbenzofuran als untere Temperaturgrenze festgelegten −30°C würden geringere Reaktionsgeschwindigkeiten mit sich bringen. Temperaturen über den erfindungsgemäß für die Umsetzung des Chlorglyoxylsäureäthylesters mit dem 2,3-Dihydro-2-äthylbenzofuran als obere Grenze festgelegten +50°C würden vom Gesichtspunkt der Ausbeuten zu keinen nennenswerten Nachteilen führen, sie können jedoch Probleme im Zusammenhang mit der Abführung der Reaktionswärme zur Folge haben. Besonders bevorzugt werden Temperaturen von −10 bis +30°C angewandt.

Vorteilhaft wird zur Umsetzung des 2,3-Dihydro-2-äthylbenzofuran-5-ylglyoxylsäureäthylesters mit dem 80 bis 100%igen Hydrazinhydrat 2 Mol bis zu einem Überschuß von 15 bis 20 Mol des letzteren je Mol des ersteren verwendet. Besonders bevorzugt werden dabei 2,25 bis 10 Mol des letzteren je Mol des ersteren eingesetzt.

Höhere Hydrazinhydratmengen führen zu einem höheren Reaktionsteilnehmerverbrauch, ohne andererseits eine nennenswerte Ausbeuteerhöhung zu ergeben.

Vorzugsweise wird bzw. werden für die Umsetzung des 2,3-Dihydro-2-äthylbenzofuran-5-ylglyoxylsäureäthylesters mit dem 80 bis 100%igen Hydrazinhydrat Temperaturen von 80 bis 200° C angewandt, wobei unter Atmosphärendruck gearbeitet werden kann.

Besonders bevorzugt wird die Umsetzung des 2,3-Dihydro-2-äthylbenzofuran-5-ylglyoxylsäureäthylesters mit dem 80 bis 100%igen Hydrazinhydrat in Äthylenglykol als Glykol durchgeführt.

Vorteilhaft wird bzw. werden beim Erhitzen des 2,3-Dihydro-2-äthylbenzofuran-5-ylglyoxylsäurehydrazidhydrazones, welche Umsetzung als Wolff-Kishner-Reaktion (Merck Index ONR 96 9. Auflage) bekannt ist, als starke anorganische Base(n) 1 oder mehr Alkalimetallhydroxyde und/oder -carbonate, insbesondere Kaliumhydroxyd, Natriumhydroxyd und/oder Kaliumcarbonat, in Lösung in Wasser, einem Alkohol, wie untenstehend definiert oder einem Glykol, in Form von Äthylenglykol oder Propylenglykol, oder einer Mischung derselben, insbesondere einer wäßrigalkoholischen Mischung, verwendet. Es ist auch bevorzugt, beim Erhitzen des 2,3-Dihydro-2-äthylbenzofuran-5-ylglyoxylsäurehydrazidhydrazones die starke(n) anorganische(n) Base(n) in einer Menge von 3 bis 6 Mol, bezogen auf 1 Mol 2,3-Dihydro-2-äthylbenzofuran-5-ylglyoxylsäureäthylester, zu verwenden.

Zweckmäßig wird auch das Erhitzen des 2,3-Dihydro-2-äthylbenzofuran-5-ylglyoxylsäurehydrazidhydrazones (Wolff-Kishner-Reaktion) in 1 oder mehr Alkoholen mit 1 bis 6 Kohlenstoffatomen und/oder den oben bezeichneten Glykolen durchgeführt. Dabei werden als Alkohole bevorzugt solche mit 4 bis 6 Kohlenstoffatomen verwendet, da mit ihnen der Vorteil verbunden ist, daß unter Atmosphärendruck gearbeitet werden kann. Als Glykol wird vorzugsweise Äthylenglykol oder Propylenglykol verwendet.

Nach einer Variante des erfindungsgemäßen Verfahrens wird der 2,3-Dihydro-2-äthylbenzofuran-5-ylglyoxylsäureäthylester zunächst mit 1 bis 2 Mol 80 bis 100%igem Hydrazinhydrat in 1 oder mehr aliphatischen Alkoholen mit 1 bis 4 Kohlenstoffatomen als Lösungsmittel(n) bei Temperaturen von −5 bis +40°C zu 2,3-Dihydro-2-äthylbenzofuran-5-ylglyoxylsäurehydrazid umgesetzt und das letztere aus der Reaktionsmischung isoliert und anschließend mit 1,25 bis 5 Mol 80 bis 100%igem Hydrazinhydrat zum 2,3-Dihydro-2-äthylbenzofuran-5-ylglyoxylsäurehydrazidhydrazon weiter umgesetzt. Dann erfolgt das Erhitzen des lezteren in der oben angegebenen Weise.

Zweckmäßig wird bei dieser Variante des erfindungsgemäßen Verfahrens in der Weise vorgegangen, daß der aus der Reaktionsmischung der Friedel-Crafts-Umsetzung, das heißt der Umsetzung des Chlorglyoxylsäureäthylesters mit dem 2,3-Dihydro-2-äthylbenzofuran durch Entfernung des Lösungsmittels erhaltene rohe 2,3-Dihydro-2-äthylbenzofuran-5-ylglyoxysäureäthylester ohne Reinigung durch Destillation mit den 1 bis 2 Mol 80 bis 100%igem Hydrazinhydrat bei einer Temperatur von −5 bis +40°C zum entsprechenden Hydrazid 2,3-Dihydro-2-äthylbenzofuran-5-ylglyoxylsäurehydrazid umgesetzt wird. Die Isolierung des so erhaltenen 2,3-Dihydro-2-äthylbenzofuran-5-ylglyoxylsäurehydrazides kann vorteilhaft durch Kristallisieren erfolgen.

Die sonstigen Reaktionsbedingungen, unter welchen diese Variante des erfindungsgemäßen Verfahrens durchgeführt werden kann, sind den oben für die anderen Ausführungsformen des erfindungsgemäßen Verfahrens angegebenen im wesentlichen gleich.

In jedem Falle kann die 2,3-Dihydro-2-äthylbenzofuran-5-ylessigsäure aus der sie enthaltenden Reaktionsendmischung wie folgt isoliert werden: Diese Reaktionsmischung wird angesäuert und der erhaltene Niederschlag wird durch Filtrieren abgetrennt oder mit einem organischen Lösungsmittel extrahiert. Eine weitere Reinigung kann durch Lösen in einem eine organische oder anorganische Base enthaltenden wäßrigen Medium unter Erzielung eines Salzes, welches gegebenenfalls isoliert werden kann, und Rückverwandlung desselben zur 2,3-Dihydro-2-äthylbenzofuran-5-ylessigsäure erfolgen.

Ein sehr bedeutender Vorteil des erfindungsgemäßen Verfahrens gegenüber den Verfahren des Standes der Technik liegt in der beträchtlichen Erhöhung der Ausbeute am gewünschten Produkt 2,3-Dihydro-2-äthylbenzofuran-5-ylessigsäure. Die Ausbeute kann nämlich beim erfindungsgemäßen Verfahren auf 65,6% gegenüber 46,4%, jeweils bezogen auf das eingesetzte 2,3-Dihydro-2-äthylbenzofuran, beim genannten bekannten Verfahren erhöht sein, was einer prozentualen Ausbeuteerhöhung von etwa 41% entspricht.

Ein anderer wesentlicher Vorteil des erfindungsgemäßen Verfahrens rührt von der Verwendung von Chlorkohlenwasserstofflösungsmitteln bei der Umsetzung des Chlorglyoxylsäureäthylesters mit dem 2,3-Dihydro-2-äthylbenzofuran, welche durch einfaches Abdampfen entweder unter Atmosphärendruck oder unter Vakuum ohne die Nachteile des Standes der Technik leicht entfernt werden können, her. Außerdem sind die erfindungsgemäß verwendeten Lösungsmittel weniger toxisch als das nach dem genannten bekannten Verfahren verwendete Nitrobenzol. So sind vom Methylenchlorid, einem der erfindungsgemäß bevorzugt verwendeten Lösungsmittel, in Arbeitsräumen 100fache Höchstkonzentrationen im Vergleich zum Nitrobenzol zulässig.

Die Verwendung des Titantetrachlorides und/oder Zinntetrachlorides als Friedel-Crafts-Katalysator im erfindungsgemäßen Verfahren bringt den großen Vorteil mit sich, daß sie die Durchführung der Friedel-Crafts-Reaktion (Umsetzung des Chlorglyoxylsäureäthylesters mit dem 2,3-Dihydro-2-äthylbenzofuran) in einem ziemlich weiten Temperaturbereich gestattet, ohne daß sich die Bildung von unerwünschten Nebenprodukten zeigt.

Die Löslichkeit des im erfindungsgemäßen Verfahren als Friedel-Crafts-Katalysator verwendeten Titantetrachlorides bzw. Zinntetrachlorides im Reaktionsmedium stellt einen weiteren bedeutenden Vorteil dar. Wie es nämlich bekannt ist, hat das nach dem genannten bekannten Verfahren als Friedel-Crafts-Katalysator verwendete Aluminiumchlorid eine verhältnismäßig geringe Löslichkeit im Nitrobenzol und daher ist es, wenn die Durchführung der Umsetzung in homogener Phase gewünscht wird, notwendig, beträchtliche Mengen dieses Lösungsmittels einzusetzen. Wenn nach dem genannten Stand der Technik als andere Möglichkeit die Friedel-Crafts-Reaktion in Gegenwart einer geringeren Nitrobenzolmenge durchgeführt wird, muß mit einer Suspension des Aluminiumchlorides im Lösungsmittel gearbeitet werden. In diesem Falle ist es jedoch notwendig, ein ständiges kräftiges Rühren zur Vermeidung einer Inhomogenität der Reaktionsmischung (beispielsweise durch Absetzen bzw. Sedimentierung des Aluminiumchlorides), welche örtliche Überhitzungen mit schädlichen Folgen herbeiführen könnte, aufrechtzuerhalten. Im Gegensatz dazu gestatten im erfindungsgemäßen Verfahren das Titantetrachlorid und Zinntetrachlorid, welche Flüssigkeiten sind und sich auch in geringen Mengen der als Reaktionsmedium verwendeten Chlorkohlenwasserstoffe lösen, die leichte Verwirklichung eines homogenen Reaktionsmediums, in welchem die Verteilung der Reaktionswärme wirksam erfolgen kann, ohne örtliche Überhitzungen zu riskieren.

Ein weiterer wesentlicher Vorteil des erfindungsgemäßen Verfahrens besteht darin, daß der bei der Friedel-Crafts-Reaktion erhaltene 2,3-Dihydro-2-äthylbenzofuran-5-ylglyoxylsäureäthylester unmittelbar der Umsetzung mit dem Hydrazinhydrat und anschließenden alkalischen Zersetzung (Wolff-Kishner-Reaktion) unterzogen werden kann, ohne daß eine vorherige Hydrolyse zur freien Säure, wie sie nach dem Stand der Technik zu erfolgen hat, erforderlich wäre.

Das erfindungsgemäße Verfahren einschließlich der Stufe der Umsetzung des Chlorglyoxylsäureäthylesters mit dem 2,3-Dihydro-2-äthylbenzofuran in im wesentlichen äquimolaren Mengen in 1 oder mehr Chlorkohlenwasserstoffen als Lösungsmittel(n) in Gegenwart von Titantetrachlorid und/oder Zinntetrachlorid ist auch unter Berücksichtigung der Druckschrift Journal of the Chemical Society 1952, Seiten 376 bis 380 sehr überraschend. Insbesondere ergibt sich dies aus der dort stehenden Angabe, daß Titantetrachlorid als Friedel-Crafts-Katalysator weniger wirksam als Aluminiumchlorid ist, zumal in Verbindung mit der Tatsache, daß die Ausbeuten an den Acylierungsprodukten gering sind. Gegenüber der aus dieser Angabe sich ergebenden Erwartung des Durchschnittsfachmannes, daß mit Titantetrachlorid als Friedel-Crafts-Katalysator schlechtere Ausbeuten als die 60,3% an 2,3-Dihydro-2-äthylbenzofuran-5-ylglyoxylsäureäthylester, bezogen auf das eingesetzte 2,3-Dihydro-2-äthylbenzofuran, und damit als die 46,4% an 2,3-Dihydro-2-äthylbenzofuran-5-ylessigsäure, bezogen auf das eingesetzte 2,3-Dihydro-2-äthylbenzofuran, beim Verfahren der britischen Patentschrift

**0 008 752**

1 483 061 erhalten werden müßten, ist es sehr überraschend, daß im erfindungsgemäßen Verfahren mit Titantetrachlorid als Friedel-Crafts-Katalysator gerade im Gegenteil die höheren Ausbeuten von 77,2% an 2,3-Dihydro-2-äthylbenzofuran-5-ylglyoxylsäureäthylester, bezogen auf das eingesetzte 2,3-Dihydro-2-äthylbenzofuran, und damit 65,6% an 2,3-Dihydro-2-äthylbenzofuran-5-ylessigsäure, bezogen auf das eingesetzte 2,3-Dihydro-2-äthylbenzofuran, erzielt werden können. Auch ist gegenüber der Angabe der mangelnden Reinheit der nach dem Verfahren der Druckschrift Journal of the Chemical Society 1952, Seiten 376 bis 380 erhaltenen Acylierungsprodukte wegen deren Verunreinigung mit Teeren die hohe Reinheit der nach dem erfindungsgemäßen Verfahren erhaltenen 2,3-Dihydro-2-äthylbenzofuran-5-ylessigsäure überraschend. Ferner war auch die aus der Druckschrift Journal of the Chemical Society 1952, Seiten 376 bis 380 hervorgehende Verwendung der zu acylierenden Verbindungen im Überschuß gegenüber den Acylierungsmitteln geeignet, den Fachmann vom erfindungsgemäßen Verfahren, in welchem die zu acylierende Verbindung 2,3-Dihydro-2-äthylbenzofuran und das Acylierungsmittel Chlorglyoxylsäureäthylester in im wesentlichen äquimolaren Mengen verwendet werden, abzulenken.

Die Erfindung wird an Hand der folgenden Beispiele näher erläutert.

### Beispiel 1

#### a) Herstellung von 2,3-Dihydro-2-äthylbenzofuran-5-ylglyoxylsäureäthylester

Es wurde zu einer Lösung von 635,5 g (3,25 Mol) Titantetrachlorid in 1250 cm³ wasserfreiem Methylenchlorid während 3 Stunden bei einer Temperatur von −5 bis 0°C eine Mischung von 474,3 g (3,20 Mol) 2,3-Dihydro-2-äthylbenzofuran und 443 g (3,25 Mol) Chlorglyoxylsäureäthylester zugegeben. Nach 3 Stunden langem Rühren bzw. Schütteln bei einer Temperatur von 0 bis 5°C wurde die Mischung in 1500 cm³ einer eiskalten 6 n Salzsäure eingegossen, wobei dafür gesorgt wurde, daß die Innentemperatur 10°C nicht überstieg.

Die organische Phase wurde abgetrennt, mit einer 3 n Salzsäure und Wasser gewaschen, über wasserfreiem Natriumsulfat getrocknet und unter Vakuum eingedampft. Es wurden 785 g eines öligen Rückstandes, bestehend aus rohem 2,3-Dihydro-2-äthylbenzofuran-5-ylglyoxylsäureäthylester, erhalten. Aus diesem wurden durch Destillation unter vermindertem Druck als Produkt 613,6 g (77,2% der Theorie) reiner 2,3-Dihydro-2-äthylbenzofuran-5-ylglyoxylsäureäthylester mit einem Siedepunkt von 193 bis 195°C (2 mm Hg) erhalten.

#### b) Herstellung von 2,3-Dihydro-2-äthylbenzofuran-5-ylessigsäure

Es wurden zu 750 cm³ (13,1 Mol) 85%igem Hydrazinhydrat bei 60 bis 80°C 496,6 g (2,0 Mol) 2,3-Dihydro-2-äthylbenzofuran-5-ylglyoxylsäureäthylester zugegeben und es wurde auf 110°C erhitzt. Nach 1 Stunde langem Erhitzen auf 110°C wurde allmählich während 60 Minuten eine Lösung von 360,0 g (5,5 Mol) Kaliumhydroxyd in 600 cm³ Äthylenglykol zugegeben, wobei die Temperatur allmählich auf 130°C gebracht wurde. Nach 2 Stunden langem Erhitzen auf diese Temperatur wurde unter 100°C gekühlt, mit 400 cm³ Wasser verdünnt und mit konzentrierter Salzsäure angesäuert. Der Niederschlag wurde gesammelt, mit Wasser gewaschen und in 1000 cm³ einer 3 n Natriumhydroxydlösung gelöst. Die Lösung wurde mit Methylenchlorid gewaschen, mit einer 6 n Salzsäure angesäuert und mit 1000 cm³ Methylenchlorid extrahiert. Der Methylenchloridauszug wurde über wasserfreiem Natriumsulfat getrocknet und unter Vakuum eingedampft. Der Rückstand wurde aus einer Mischung aus 350 cm³ Tetrachlorkohlenstoff und 700 cm³ Cyclohexan kristallisiert. So wurden 350 g 2,3-Dihydro-2-äthylbenzofuran-5-ylessigsäure erhalten. Dies entspricht einer Ausbeute von 85,0% der Theorie, bezogen auf den eingesetzten 2,3-Dihydro-2-äthylbenzofuran-5-ylglyoxylsäureäthylester, und einer Gesamtausbeute von 65,6% der Theorie, bezogen auf das als Ausgangsstoff eingesetzte 2,3-Dihydro-2-äthylbenzofuran.

### Beispiel 2

Es wurde wie im Beispiel 1, Abschnitt a) beschrieben gearbeitet, wobei dieselben Substanzmengen eingesetzt wurden. Der ölige Rückstand, bestehend aus 2,3-Dihydro-2-äthylbenzofuran-5-ylglyoxylsäureäthylester, wurde in 2300 cm³ Methanol gelöst und diese Lösung wurde mit 270 cm³ (4,7 Mol) 85%igem Hydrazinhydrat bei 10 bis 20°C behandelt. Beim Stehen über Nacht bei 5°C kristallisierten 551,8 g 2,3-Dihydro-2-äthylbenzofuran-5-yloxalylhydrazid mit einem Schmelzpunkt von 128 bis 130°C. Die Analysenwerte (C,H, N) und die Spektraldaten (UV-und Ultrarotspektren) waren mit seiner Formel in Übereinstimmung. Die Ausbeute betrug 73,6% der Theorie, bezogen auf das als Ausgangsstoff eingesetzte 2,3-Dihydro-2-äthylbenzofuran.

5

Dann wurden 504,5 g (2,0 Mol) des wie vorstehend beschrieben erhaltenen 2,3-Dihydro-2-äthylbenzofuran-5-yloxalylhydrazides zu einer Lösung von 353 cm³ (6,2 Mol) 85%igem Hydrazinhydrat in 600 cm³ Äthylenglykol zugegeben und es wurde auf 110°C erhitzt. Im übrigen wurde wie im Beispiel 1 beschrieben vorgegangen.

So wurden 350 g 2,3-Dihydro-2-äthylbenzofuran-5-ylessigsäure erhalten. Dies entspricht einer Ausbeute von 85,0% der Theorie, bezogen auf das eingesetzte 2,3-Dihydro-2-äthylbenzofuran-5-yloxalylhydrazid, und einer Gesamtausbeute von 62,6% der Theorie, bezogen auf das als Ausgangsstoff eingesetzte 2,3-Dihydro-2-äthylbenzofuran.

## Beispiel 3

Es wurde wie im Beispiel 1, Abschnitt a) beschrieben mit denselben Substanzmengen gearbeitet, jedoch mit dem Unterschied, daß als Friedel-Crafts-Katalysator 872,7 g (3,35 Mol) Zinntetrachlorid verwendet wurden und die Reaktionsmischung statt 3 Stunden über Nacht bei einer Temperatur von 0 bis 5°C gerührt bzw. geschüttelt wurde.

So wurden 554,3 g 2,3-Dihydro-2-äthylbenzofuran-5-yl-glyoxylsäureäthylester erhalten. Dies entspricht einer Ausbeute von 69,8% der Theorie, bezogen auf das als Ausgangsstoff eingesetzte 2,3-Dihydro-2-äthylbenzofuran.

Dann wurde der erhaltene 2,3-Dihydro-2-äthylbenzofuran-5-ylglyoxylsäureäthylester nach der im Beispiel 1, Abschnitt b) beschriebenen Verfahrensweise weiter umgesetzt. So wurde 2,3-Dihydro-2-äthylbenzofuran-5-ylessigsäure in einer Gesamtausbeute von 59,3% der Theorie, bezogen auf das als Ausgangsstoff eingesetzte 2,3-Dihydro-2-äthylbenzofuran, erhalten.

## Beispiel 4

Es wurde bis zum Ansäuern mit konzentrierter Salzsäure wie im Beispiel 1 beschrieben vorgegangen. Der so erhaltene Niederschlag wurde mit 1000 cm³ Methylenchlorid extrahiert und die organische Phase wurde mit Wasser gewaschen und zur Trockne eingedampft. Der Rückstand wurde in 600 cm³ Aceton gelöst und die Lösung wurde mit 362,6 g (2,0 Mol) Dicyclohexylamin behandelt. Nach dem Stehenlassen über Nacht bei +5°C wurden auf dem Filter 668,9 g (86,3% der Theorie, bezogen auf den eingesetzten 2,3-Dihydro-2-äthylbenzofuran-5-ylglyoxysäureäthylester) des Dicyclohexylaminsalzes der 2,3-Dihydro-2-äthylbenzofuran-5-ylessigsäure gesammelt. Es wurde mit Aceton gewaschen und getrocknet.

Das Dicyclohexylaminsalz der 2,3-Dihydro-2-äthylbenzofuran-5-ylessigsäure wurde mit einer Mischung aus 2000 cm³ Wasser und 1000 cm³ Äthylacetat behandelt. Es wurde mit einer 30%igen Natriumhydroxydlösung alkalisch gemacht und die beiden gebildeten Phasen wurden getrennt. Die wäßrige Phase wurde mit 300 cm³ Tetrachlorkohlenstoff und 600 cm³ Cyclohexan behandelt und mit konzentrierter Salzsäure angesäuert. Der gefällte feste Stoff wurde nach dem Stehenlassen über Nacht bei +5°C auf einem Filter gesammelt und mit Wasser gewaschen.

So wurden 320,1 g (77,6% der Theorie) 2,3-Dihydro-2-äthylbenzofuran-5-ylessigsäure, bezogen auf den eingesetzten 2,3-Dihydro-2-äthylbenzofuran-5-ylglyoxylsäureäthylester, erhalten. Dies entspricht einer Gesamtausbeute von 59,9% der Theorie, bezogen auf das als Ausgangsstoff eingesetzte 2,3-Dihydro-2-äthylbenzofuran.

## Vergleichsbeispiel

### nach GB-A-1 483 061, Verfahren 1b)

Es wurde zu einer Suspension von 446,7 g (3,35 Mol) wasserfreiem Aluminiumtrichlorid in 1800 cm³ Nitrobenzol während 3 Stunden unter Rühren beziehungsweise Schütteln und Halten der Temperatur auf −5 bis −2°C eine Mischung von 474,3 g (3,20 Mol) 2,3-Dihydro-2-äthylbenzofuran und 443,8 g (3,25 Mol) Chlorglyoxylsäureäthylester zugegeben. Nach weiteren 3 Stunden bei −5 bis −2°C wurde die Mischung in 1500 cm³ einer eiskalten 6 n Salzsäure eingegossen, wobei von außen gekühlt wurde. Die organische Phase wurde abgetrennt und mit einer 3 n Salzsäure und dann mit Wasser gewaschen und schließlich wurde das Nitrobenzol durch Wasserdampfdestillation entfernt. Der Rückstand wurde unter Vakuum destilliert, wobei 479,0 g (60,3% der Theorie) 2,3-Dihydro-2-äthylbenzofuran-5-ylglyoxylsäureäthylester mit einem Siedepunkt von 193 bis 195°C (2 mm Hg) aufgefangen wurden.

Dann wurde eine Suspension des wie vorstehend beschrieben erhaltenen 2,3-Dihydro-2-äthylbenzofuran-5-ylglyoxylsäureäthylester in einer Mischung von 1000 cm³ Äthylalkohol und 1000 cm³ Wasser mit einem Gehalt an 100 g (2,5 Mol) Natriumhydroxyd 45 Minuten lang unter Rückfluß zum Sieden erhitzt. Die mit einer 6 n Salzsäure angesäuerte Lösung ergab 384,5 g 2,3-Dihydro-2-äthylbenzofuran-5- ylglyoxylsäure, welche bei der anschließenden Umsetzung ohne weitere Reinigung unmittelbar verwendet werden konnte. Die Ausbeute der Hydrolyse betrug 90,5% der Theorie.

Daraufhin wurden 440,5 g (2,0 Mol) der wie vorstehend beschrieben erhaltenen 2,3-Dihydro-2-äthyl-benzofuran-5-ylglyoxylsäure zu 750,0 cm$^3$ (13,1 Mol) 85%igem Hydrazinhydrat zugegeben und es wurde 1 Stunde lang auf 110°C erhitzt. Dann wurde während 60 Minuten allmählich eine Lösung von 360,0 g (5,5 Mol) Kaliumhydroxyd in 600 cm$^3$ Äthylenglykol zugegeben und gleichzeitig wurde die Temperatur allmählich auf 130°C erhöht. Nach 2 Stunden langem Erhitzen auf 130°C wurde unter 100°C gekühlt, mit 400 cm$^3$ Wasser verdünnt und mit konzentrierter Salzsäure angesäuert. Der Niederschlag wurde gesammelt und wie im Beispiel 1 beschrieben aufgearbeitet. So wurden 350,6 g 2,3-Dihydro-2-äthylbenzofuran-5-ylessigsäure erhalten. Dies entspricht einer Ausbeute von 85,0% der Theorie, bezogen auf die eingesetzte 2,3-Dihydro-2-äthylbenzofuran-5-ylglyoxylsäure, und einer Gesamtausbeute von 46,4% der Theorie, bezogen auf das als Ausgangsstoff eingesetzte 2,3-Dihydro-2-äthylbenzofuran.

Die als Produkt des erfindungsgemäßen Verfahrens erhaltene 2,3-Dihydro-2-äthylbenzofuran-5-ylessigsäure kann in der Therapie als entzündungshemmendes Mittel verwendet werden.

**Patentansprüche**

1. Verfahren zur Herstellung von 2,3-Dihydro-2-äthylbenzofuran-5-ylessigsäure durch Umsetzen von im wesentlichen äquimolaren Mengen von Chlorglyoxylsäureäthylester und 2,3-Dihydro-2-äthylbenzo-furan in Gegenwart eines Friedel-Crafts-Katalysators in einem Lösungsmittel bei niederer Temperatur sowie im weiteren unter Verwendung einer Umsetzung mit Hydrazinhydrat als Reaktionsteilnehmer, dadurch gekennzeichnet, daß man für die Umsetzung des Chlorglyoxylsäureäthylesters mit dem 2,3-Dihydro-2-äthylbenzofuran als Friedel-Crafts-Katalysator Titantetrachlorid und/oder Zinntetrachlorid und als Lösungsmittel 1 oder mehr niedere aliphatische Chlorkohlenwasserstoffe einsetzt und als Temperatur −30 bis +50°C anwendet sowie den aus der Reaktionsmischung isolierten erhaltenen 2,3-Dihydro-2-äthylbenzofuran-5-ylglyoxylsäureäthylester unmittelbar mit 80 bis 100%igem Hydrazinhydrat in Gegenwart von 1 oder mehr Alkoholen mit 1 bis 6 Kohlenstoffatomen und/oder Glykolen mit 2 bis 4 Kohlenstoffatomen als Lösungsmittel zum 2,3-Dihydro-2-äthylbenzofuran-5-ylglyoxylsäurehydra-zidhydrazon umsetzt und das letztere unmittelbar in Gegenwart einer Lösung von 1 oder mehr starken anorganischen Basen auf eine Temperatur von 100 bis 200°C erhitzt.

2. Verfahren nach Anspruch 1, dadurch gekennzeichnet, daß man für die Umsetzung des Chlorglyoxylsäureäthylesters mit dem 2,3-Dihydro-2-äthylbenzofuran einen leichten Überschuß des Chlorglyoxylsäureäthylesters von 1,05 bis 1,3 Mol je Mol 2,3-Dihydro-2-äthylbenzofuran verwendet.

3. Verfahren nach Anspruch 1 oder 2, dadurch gekennzeichnet, daß man für die Umsetzung des Chlorglyoxylsäureäthylesters mit dem 2,3-Dihydro-2-äthylbenzofuran den Friedel-Crafts-Katalysator in einer Menge von 1 bis 1,2 Mol je Mol 2,3-Dihydro-2-äthylbenzofuran verwendet.

4. Verfahren nach Anspruch 1 bis 3, dadurch gekennzeichnet, daß man für die Umsetzung des Chlorglyoxylsäureäthylesters mit dem 2,3-Dihydro-2-äthylbenzofuran als Lösungsmittel Methylenchlorid, Chloroform, Tetrachlorkohlenstoff, Dichloräthan oder Tetrachloräthan oder eine Mischung derselben verwendet.

5. Verfahren nach Anspruch 1 bis 4, dadurch gekennzeichnet, daß man für die Umsetzung des Chlorglyoxylsäureäthylesters mit dem 2,3-Dihydro-2-äthylbenzofuran Temperaturen von −10 bis +30°C anwendet.

6. Verfahren nach Anspruch 1 bis 5, dadurch gekennzeichnet, daß man für die Umsetzung des 2,3-Dihydro-2-äthylbenzofuran-5-ylglyoxylsäureäthylesters mit dem 80- bis 100%igen Hydrazinhydrat 2,25 bis 10 Mol des letzteren je Mol des ersteren einsetzt.

7. Verfahren nach Anspruch 1 bis 6, dadurch gekennzeichnet, daß man für die Umsetzung des 2,3-Dihydro-2-äthylbenzofuran-5-ylglyoxylsäureäthylesters mit dem 80- bis 100%igen Hydrazinhydrat Temperaturen von 80 bis 200°C anwendet.

8. Verfahren nach Anspruch 1 bis 6, dadurch gekennzeichnet, daß man die Umsetzung des 2,3-Dihydro-2-äthylbenzofuran-5-ylglyoxylsäureäthylesters mit dem 80- bis 100%igen Hydrazinhydrat in Äthylenglykol durchführt.

9. Verfahren nach Anspruch 1 bis 8, dadurch gekennzeichnet, daß man beim Erhitzen des 2,3-Dihydro-2-äthylbenzofuran-5-ylglyoxylsäurehydrazidhydrazones als starke anorganische Base(n) Kaliumhydroxyd, Natriumhydroxyd und/oder Kaliumcarbonat in Lösung in Wasser, einem Alkohol mit 1−6 Kohlenstoffatomen oder einem Äthylen- bzw. Propylenglykol oder einer Mischung derselben verwendet.

10. Verfahren nach Anspruch 1 bis 9, dadurch gekennzeichnet, daß man beim Erhitzen des 2,3-Dihydro-2-äthylbenzofuran-5-ylglyoxylsäurehydrazidhydrazones die starke(n) anorganische(n) Base(n) in einer Menge von 3 bis 6 Mol, bezogen auf 1 Mol 2,3-Dihydro-2-äthylbenzofuran-5-ylglyoxyl-säureäthylester, verwendet.

11. Verfahren nach Anspruch 1 bis 5, 9 oder 10, dadurch gekennzeichnet, daß man den 2,3-Dihydro-2-äthylbenzofuran-5-ylglyoxylsäureäthylester zunächst mit 1 bis 2 Mol 80- bis 100%igem

0 008 752

Hydrazinhydrat in 1 oder mehr aliphatischen Alkoholen mit 1 bis 4 Kohlenstoffatomen als Lösungsmittel(n) bei Temperaturen von $-5$ bis $+40°$ C zu 2,3-Dihydro-2-äthylbenzofuran-5-ylglyoxyl-säurehydrazid umsetzt und das letztere aus der Reaktionsmischung isoliert und mit 1,25 bis 5 Mol 80- bis 100%igem Hydrazinhydrat zum 2,3-Dihydro-2-äthylbenzofuran-5-ylglyoxylsäurehydrazidhydrazon weiter umsetzt.

## Claims

1. A process for preparing 2,3-dihydro-2-ethylbenzofurane-5-ylacetic acid by reacting essentially equimolar quantities of chloroglyoxylic acid ethylester and 2,3-dihydro-2-ethylbenzofurane in the presence of a Friedel-Crafts catalyst in a solvent at low temperature as well as in the further course making use of a reaction with hydrazine hydrate as reactant, characterized in that one uses for the reaction of the chloroglyoxylic acid ethylester with the 2,3-dihydro-2-ethylbenzofurane as Friedel-Crafts catalyst titanium tetrachloride and/or tin tetrachloride an as a solvent 1 or more lower aliphatic chlorinated hydrocarbons and one applies as temperature form $-30$ to $+50°$ C as well as one makes to react the obtained 2,3-dihydro-2-ethylbenzofurane-5-ylglyoxylic acid ethylester isolated from the reaction mixture immediately with 80 to 100% hydrazine hydrate in the presence of 1 or more alcohols having from 1 to 6 carbon atoms and/or glycoles having from 2 to 4 carbon atoms as soltent(s) to yield 2,3-dihydro-2-ethylbenzofurane-5-ylglyoxylic acid hydrazide hydrazone and one heats the latter immediately in the presence of a solution of 1 or more strong inorganic bases to a temperature of 100 to 200° C.

2. A process according to claim 1, characterized in that one uses for the reaction of the chloroglyoxylic acid ethylester with the 2,3-dihydro-2-ethylbenzofurane a small excess of the chloroglyoxylic acid ethylester of from 1.05 to 1.3 mole per mole of 2,3-dihydro-2-ethylbenzofurane.

3. A process according to claim 1 or 2, characterized in that one uses the Friedel-Crafts catalyst for the reaction of the chloroglyoxylic acid ethylester with the 2,3-dihydro-2-ethylbenzofurane in an amount of from 1 to 1.2 mole per mole of 2,3-dihydro-2-ethylbenzofurane.

4. A process according to claim 1 to 3, characterized in that one uses as solvent for the reaction of the chloroglyoxylic acid ethylester with the 2,3-dihydro-2-ethylbenzofurane methylene chloride, chloroform, carbon tetrachloride, dichloroethane or tetrachloroethane or a mixture of them.

5. A process according to claims 1 to 4, characterized in that one applies for the reaction of the chloroglyoxylic acid ethylester with the 2,3-dihydro-2-ethylbenzofurane temperatures of from $-10$ to $+30°$ C.

6. A process according to claims 1 to 5, characterized in that one uses for the reaction of the 2,3-dihydro-2-ethylbenzofurane-5-ylglyoxylic acid ethylester with the 80 to 100% hydrazine hydrate from 2.25 to 10 mole of the latter per mole of the former.

7. A process according to claims 1 to 6, characterized in that one applies for the reaction of the 2,3-dihydro-2-ethylbenzofurane-5-ylglyoxylic acid ethylester with the 80 to 100% hydrazine hydrate temperatures of from 80 to 200° C.

8. A process according to claims 1 to 6, characterized in that one carries out the reaction of the 2,3-dihydro-2-ethylbenzofurane-5-ylglyoxylic acid ethylester with the 80 to 100% hydrazine hydrate in ethylene glycol.

9. A process according to claims 1 to 8, characterized in that one uses as strong inorganic base(s) when heating the 2,3-dihydro-2-ethylbenzofurane-5-ylglyoxylic acid hydrazide hydrazone potassium hydroxide, sodium hydroxide and/or potassium carbonate in solution in water, an alcohol having from 1 to 6 carbon atoms or an ethylene glycol or propylene glycol, respectively, or a mixture of them.

10. A process according to claims 1 to 9, characterized in that one uses the strong inorganic base(s) when heating the 2,3-dihydro-2-ethylbenzofurane-5-ylglyoxylic acid hydrazide hydrazone in an amount of from 3 to 6 moles, calculated on 1 mole of 2,3-dihydro-2-ethylbenzofurane-5-ylglyoxylic acid ethylester.

11. A process according to claims 1 to 5, 9 or 10, characterized in that one first makes to react the 2,3-dihydro-2-ethylbenzofurane-5-ylglyoxylic acid ethylester with 1 to 2 moles of 80 to 100% hydrazine hydrate in 1 or more aliphatic alcohols having from 1 to 4 carbon atoms as solvent(s) at temperatures of from $-5$ to $+40°$ C to yield 2,3-dihydro-2-ethylbenzofurane-5-ylglyoxylic acid hydrazide and one isolates the latter from the reaction mixture and makes to further react it with from 1.25 to 5 mole of 80 to 100% hydrazine hydrate to yield 2,3-dihydro-2-ethylbenzofurane-5-ylglyoxylic acid hydrazide hydrazone.

## Revendications

1. Procédé pour la preparation de l'acide 2,3-dihydro-2-éthylbenzofurane-5-ylacétique par réaction de quantités essentiellement equimolaires de l'ester éthylique de l'acide chloroglyoxylique et de 2,3-dihydro-2-éthylbenzofurane en présence d'un catalyseur de Friedel-Crafts dans un solvant a basse

8

température ainsi que dans l'ulterieur cours en si servant d'une réaction avec de l'hydrate de hydrazine comme réactif, caractérisé en ce qu'on emploie comme catalyseur de Friedel-Crafts pour la réaction de l'ester éthylique de l'acide chloroglyoxylique avex 2,3-dihydro-2-éthylbenzofurane le tetrachlorure de titane et/ou le tetrachlorure de l'étain et on emploie comme solvant 1 ou plus chlorohydrocarbures aliphatiques inferieurs et on applique comme température −30 a +50°C ainsi que on fait réagir l'ester éthylique de l'acide 2,3-dihydro-2-éthylbenzofurane-5-ylglyoxylique obtenu isolé du mélange de réaction immédiatement avec de hydrate de hydrazine ayant un titre de 80 à 100% en présence de 1 ou plus alcools ayant 1 a 6 atomes de charbon et/ou glycols ayant 2 a 4 atomes de charbon comme solvant(s) pour donner l'hydrazone de l'hydrazide de l'acid 2,3-dihydro-2-éthylbenzofurane-5-ylglyoxylique et on chauffe cette dernière immédiatement en présence d'une solution de 1 ou plus fortes bases inorganiques a une température de 100 a 200°C.

2. Procédé selon la revendication 1, caractérisé en ce qu'on emploie pour réaction de l'ester éthylique de l'acide chloroglyoxylique avec le 2,3-dihydro-2-éthylbenzofurane un léger excès de l'ester éthylique de l'acide chloroglyoxylique de 1,05 a 1,3 moléculegrammes par molécule-gramme de 2,3-dihydro-2-éthylbenzofurane.

3. Procédé selon la revendication 1 ou 2, caractérisé en ca qu'on emploie le catalyseur de Friedel-Crafts pour la réaction de l'ester éthylique de l'acide chloroglyoxylique avec le 2,3-dihydro-2-éthylbenzofurane en une quantité de 1 a 1,2 molécule-grammes par molécule-gramme de 2,3-dihydro-2-éthylbenzofurane.

4. Procédé selon les revendications 1 à 3, caractérisé en ce qu'on emploie comme solvant pour la réaction de l'ester éthylique de l'acide chloroglyoxylique avec le 2,3-dihydro-2-éthylbenzofurane il chlorure de méthyléne, chloroforme, il tetrachlorure de charbon, dichloroéthane ou tetrachloroéthane ou un mélange de ces.

5. Procédé selon les revendications 1 à 4, caractérisé en ce qu'on applique pour la réaction de l'ester éthylique de l'acide chloroglyoxylique avec le 2,3-dihydro-2-éthylbenzofurane températures de −10 à +30°C.

6. Procédé selon les revendications 1 à 5, caractérisé en ce qu'on emploie pour la réaction de l'ester éthylique de l'acide 2,3-dihydro-2-éthylbenzofurane-5-ylglyoxylique avec l'hydrate de l'hydrazine ayant un titre de 80 à 100% 2,25 à 10 molécule-grammes de ce dernier par molécule-gramme du premier.

7. Procédé selon les revendications 1 à 6, caractérisé en ce qu'on applique pour la réaction de l'ester éthylique de l'acide 2,3-dihydro-2-éthylbenzofurane-5-ylglyoxylique avec l'hydrate de l'hydrazine ayant un titre de 80 à 100% températures de 80 à 200°C.

8. Procédé selon les revendications 1 à 6, caractérisé en ce qu'on conduit la réaction de l'ester éthylique de l'acide 2,3-dihydro-2-éthylbenzofurane-5-ylglyoxylique avec l'hydrate de l'hydrazine ayant un titre de 80 à 100% dans glycol d'éthylene.

9. Procédé selon les revendications 1 à 8, caractérisé en ce qu'en chauffant l'hydrazone de l'hydrazide de l'acide 2,3-dihydro-2-éthylbenzofurane-5-ylglyoxylique on emploie comme forte(s) base(s) inorganique(s) l'hydroxyde de potassium, l'hydroxyde de sodium et/ou le carbonate de potassium en solution dans eau, un alcool ayant 1 à 6 atomes de charbons ou un glycol d'éthylene ou propylène, respectivement, ou dans un mélange de ces.

10. Procédé selon les revendications 1 à 9, caractérisé en ce qu'en chauffant l'hydrazone de l'hydrazide de l'acide 2,3-dihydro-2-éthylbenzofurane-5-ylglyoxylique on emploie les forte(s) base(s) inoïganique(s) en une quantité de 3 à 6 molécule-grammes calculée sur 1 molécule-gramme de l'ester éthylique de l'acide 2,3-dihydro-2-éthylbenzofurane-5-ylglyoxylique.

11. Procédé selon les revendications 1 à 5, 9 ou 10, caractérisé en ce qu'on fait réagir l'ester éthylique de l'acide 2,3-dihydro-2-éthylbenzofurane-5-ylglyoxylique d'abord avec de 1 à 2 molécule-grammes de hydrate de hydrazine ayant un titre de 80 à 100% dans 1 ou plus alcools aliphatiques ayant 1 à 4 atomes de charbons comme solvant(s) à températures de −5 à +40°C pour obtenir l'hydrazide de l'acide 2,3-dihydro-2-éthylbenzofurane-5-ylglyoxylique et on isole cette dernière du mélange de réaction et l'on fait réagir ultérieurement avec de 1,25 à 5 molécule-grammes de hydrate de hydrazine ayant un titre de 80 à 100% pour obtenir l'hydrazone de l'hydrazide de l'acide 2,3-dihydro-2-éthylbenzofurane-5-ylglyoxylique.